# EUROPEAN PATENT SPECIFICATION

(11) **EP 4 746 962 B1**
(45) Date of publication and mention of the grant of the patent: **02.09.2026**
(21) Application number: 24754461.2
(22) Date of filing: 18.07.2024
(51) Int. Cl.: A61P 1/00, A61K 31/357, A61P 1/16, A61P 9/00, A61P 11/00, A61P 13/10, A61P 13/12, A61P 17/00, A61P 21/00, A61P 37/02, A61P 37/06, C07D 493/18

(54) **DIHYDROARTEMISININ DERIVATIVES AND THE TREATMENT OF FIBROTIC DISEASES**
DIHYDROARTEMISININDERIVATE UND BEHANDLUNG FIBROTISCHER ERKRANKUNGEN
DÉRIVÉS DE DIHYDROARTÉMISININE ET TRAITEMENT DE MALADIES FIBROTIQUES

(30) Priority: 19.07.2023 US 202363527771 P
(43) Date of publication of application: 27.05.2026
(73) Proprietor: Greenstone Biosciences, Inc., Palo Alto, CA 94304 (US)
(72) Inventor: WU, Joseph, C., Palo Alto, CA 94304 (US); PIPLANI, Honit, Palo Alto, CA 94304 (US); ZHANG, Hao, Palo Alto, CA 94304 (US); SHIVNARAINE, Rabindra Vishwadev, Palo Alto, CA 94304 (US); YAN, Christopher David, Palo Alto, CA 94304 (US)
(74) Representative: Mewburn Ellis LLP
(86) International application number: PCT/US2024/038591
(87) International publication number: WO 2025/019703

(56) References cited:
- WO-A1-2009/043538
- WO-A1-2022/000492
- CN-A- 1 903 191
- PINHEIRO J C ET AL: "Antimalarial activity of dihydroartemisinin derivatives againstP. falciparumresistant to mefloquine: a quantum chemical and multivariate study", JOURNAL OF MOLECULAR STRUCTURE (THEOCHEM), ELSEVIER SCIENCE PUBLISHERS B.V., AMSTERDAM, NL, vol. 572, no. 1, 4 May 2017 (2017-05-04), pages 35 - 44, XP029996253, ISSN: 0166-1280, DOI: 10.1016/S0166-1280(01)00522-X
- SOOMRO SHAHID ET AL: "Design of novel artemisinin-like derivatives with cytotoxic and anti-angiogenic properties", JOURNAL OF CELLULAR AND MOLECULAR MEDICINE, vol. 15, no. 5, 12 July 2010 (2010-07-12), RO, pages 1122 - 1135, XP093202939, ISSN: 1582-1838, DOI: 10.1111/j.1582-4934.2010.01120.x

## Description

### Technical field

This invention relates to compounds that are dihydroartemisinin derivatives and the use of these compounds for the treatment of fibrotic diseases.

### Background art

### Artemisinin, dihydroartemisinin (DHA), and artesunate

Artemisinin, (1*R*,4*S*,5*R*,8*S*,9*R*,12*S*,13*R*)-1,5,9-trimethyl-11,14,15,16-tetraoxa-tetracyclo[10.3.1.0^{4,13}.0^{8,13}]hexadecan-10-one, (3*R*,5a*S*,6*R*,8a*S*,9*R*,12*S*,12a*R*)-octahydro-3,6,9-trimethyl-3,12-epoxy-12*H*-pyrano[4,3-*j*]-1,2-benzodioxepin-10(3*H*)-one, is a well-known antimalarial agent originally extracted from the herb *Artemisia annua*. It was discovered in 1972 in China. Artemisinin-based combination therapies (ACTs, therapies using artemisinin or one of its derivatives) are now standard treatment worldwide for malaria. Dihydroartemisinin, DHA, (1*R*,4*S*,5*R*,8*S*,9*R*,10*S*,12*R*,13*R*)-1,5,9-trimethyl-11,14,15,16-tetraoxa-tetracyclo[10.3.1.0^{4,13}.0^{8,13}]hexadecan-10-ol, (3*R*,5a*S*,6*R*,8a*S*,9*R*,12*S*,12a*R*)-decahydro-3,6,9-trimethyl-3,12-epoxy-12*H*-pyrano[4,3-*j*]-1,2-benzodioxepin-10-ol, as its name suggests, is a hydrogenated derivative of artemisinin. Dihydroartemisinin is the active metabolite of all artemisinin compounds (artemisinin, artesunate, artemether, arteether, etc.) and is also available as a drug itself. It is a semi-synthetic derivative of artemisinin and is widely used as an intermediate in the preparation of other artemisinin-derived antimalarial drugs. Yu et al., "Dihydroartemisinin: A Potential Drug for the Treatment of Malignancies and Inflammatory Diseases", Front. Oncol., 11, 722331 (2021), summarize recent studies and report that dihydroartemisinin has both anticancer and anti-inflammatory effects.

Artesunate is the hemisuccinate ester of dihydroartemisinin. Unlike artemisinin, dihydroartemisinin, or artemether/arteether (the methyl/ethyl ethers of dihydroartemisinin), only artesunate has sufficient water solubility to be administered intravenously. Indeed, in the United States, artesunate is only available as a powder for reconstitution for intravenous injection, though elsewhere it is readily available for oral administration in tablet or suspension form. Artesunate is used for the treatment of malaria, though not recommended for prophylaxis because of its short biological half-life. In the United States, artesunate is being tested for the topical treatment of vulvar, vaginal, and anal intraepithelial neoplasias; and it is reported to have even been tested for treatment of COVID-19.

The structures of artemisinin, dihydroartemisinin, and artesunate are:

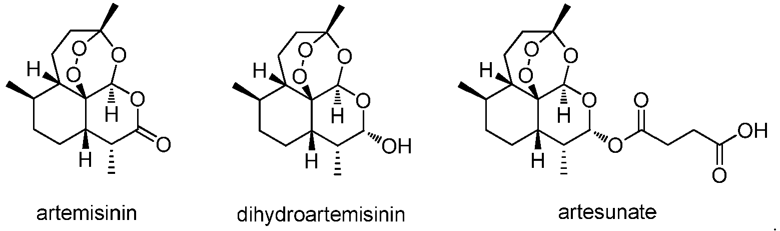

For further information on artemisinin, dihydroartemisinin, and artesunate and dihydroartemisinin, see the Wikipedia entries https://en.wikipedia.org/wiki/Artemisinin, https://en.wikipedia.org/wiki/Dihydroartemisinin, and https://en.wikipedia.org/wiki/Artesunate, and documents cited therein. A review of the pharmacokinetics of dihydroartemisinin and artesunate may be found at Morris et al., "Review of the clinical pharmacokinetics of artesunate and its active metabolite dihydroartemisinin following intravenous, intramuscular, oral or rectal administration", Malaria J., 10, 263 (2011). WO 2022/000492 describes artemisinin and derivatives thereof for treating Graves eye disease. CN 1903191 describes use of artesunate for treating hepatic fibrosis. Pinheiro et al. J. Mol. Struct. 572 (1), 2017, 35-44 reports antimalarial activity of dihydroartemisinin derivatives. WO 2009/043538 describes artemisinin derivatives for treating melanoma. Soomro et al. J. Cell Mol. Med. 2011 May;15(5), 1122-35 describes artemisinin-like derivatives with cytotoxic and anti-angiogenic properties.

### Fibrotic diseases

Rosenbloom et al., "Human Fibrotic Diseases: Current Challenges in Fibrosis Research", Fibrosis, 1627, 1-23 (2017), say: "Human fibrotic diseases constitute a major health problem worldwide owing to the large number of affected individuals, the incomplete knowledge of the fibrotic process pathogenesis, the marked heterogeneity in their etiology and clinical manifestations, the absence of appropriate and fully validated biomarkers, and, most importantly, the current void of effective disease-modifying therapeutic agents." Rosenbloom et al. list, in **Table 1,** under the category of systemic fibrotic diseases, the following: systemic sclerosis, multifocal fibrosclerosis (IgG4-associated fibrosis), nephrogenic systemic fibrosis, and sclerodermatous graft-versus-host disease; and, under the category of organ-specific fibrotic diseases, the following: cardiac fibrosis, including hypertension-associated cardiac fibrosis, post-myocardial infarction, and Chagas disease-induced myocardial fibrosis; kidney fibrosis, including diabetic and hypertensive nephropathy, urinary tract obstruction-induced kidney fibrosis, inflammatory/autoimmune-induced kidney fibrosis, aristolochic acid nephropathy, and polycystic kidney disease; pulmonary fibrosis, including idiopathic pulmonary fibrosis, silica-induced pneumoconiosis (silicosis), asbestos-induced pulmonary fibrosis (asbestosis), and chemotherapeutic agent-induced pulmonary fibrosis; liver and portal vein fibrosis, including alcoholic and nonalcoholic liver fibrosis, hepatitis C-induced liver fibrosis, primary biliary cholangitis, parasite-induced liver fibrosis (schistosomiasis); and other organ-specific fibrotic diseases, including radiation-induced fibrosis (various organs), bladder fibrosis, intestinal fibrosis, peritoneal sclerosis, diffuse fasciitis, localized scleroderma, keloids, Dupuytren's disease, Peyronie's disease, myelofibrosis, and oral submucous fibrosis. Other fibrotic diseases include cardiomyopathies such as diabetes-induced cardiomyopathy, dilated cardiomyopathy, hypertrophic cardiomyopathy, and radiation-induced cardiomyopathy, Hermansky-Pudlak syndrome, pancreatic fibrosis, the pulmonary complications of COVID-19 infection, and Duchenne muscular dystrophy.

Rosenbloom et al. continue by saying of the fibrotic diseases: "Although their causative mechanisms are quite diverse and in several instances have remained elusive, these diseases share the common feature of an uncontrolled and progressive accumulation of fibrotic tissue in affected organs causing their dysfunction and ultimate failure. Despite the remarkable heterogeneity in the etiologic mechanisms responsible for the development of fibrotic diseases and in their clinical manifestations, numerous studies have identified activated myofibroblasts as the common cellular element ultimately responsible for the replacement of normal tissues with nonfunctional fibrotic tissue", and "Although substantial progress has been accomplished regarding the pathogenesis of fibrotic diseases, they still remain a major challenge, not only because of the variety and multiplicity of initiating events but also because of the large number of profibrotic mediators involved. While TGF-β [transforming growth factor-β] is considered the primary fibrotic effector, many other cytokines and signaling molecules are involved in fibrotic reactions creating a highly complex network of redundant signaling pathways that must be considered when attempting to develop effective anti-fibrotic therapies." They also note that: "Currently, therapeutic interventions for the fibrotic diseases are quite limited. For example, only two drugs, pirfenidone and nintedanib, have been approved for IPF, and no disease-modifying drug has been approved for SSc [systemic sclerosis] or other fibrotic diseases."

Artesunate has been shown to be active as an antifibrotic agent in the bleomycin-induced pulmonary fibrosis model in rats: *see* Wang et al., "Anti-profibrotic effects of artesunate on bleomycin-induced pulmonary fibrosis in Sprague Dawley rats", Mol. Med. Rep., 12, 1291-1297 (2015), and Liu et al., "Artesunate ameliorates lung fibrosis via inhibiting the Notch signaling pathway", Exp. Ther. Med., 14, 561-1566 (2017). However, the short biological half-life of artesunate renders it unsuitable as a long-term drug, particularly an oral drug, for the treatment of fibrotic diseases.

It would be desirable to develop new dihydroartemisinin derivatives as pharmacological treatments for fibrotic diseases.

### Summary of the invention

In a first aspect, this invention is compounds of any one of the formulae PT-1, PT-2, PT-4, PT-13, PT-14, PT-20, PT-23 to PT-25, and PT-27 to PT-30: 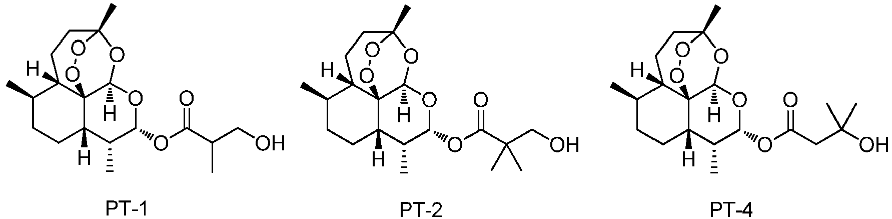 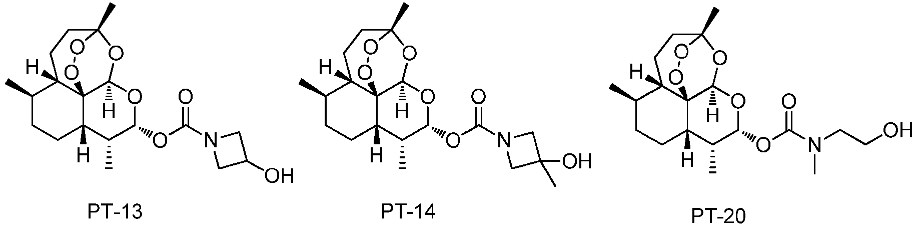 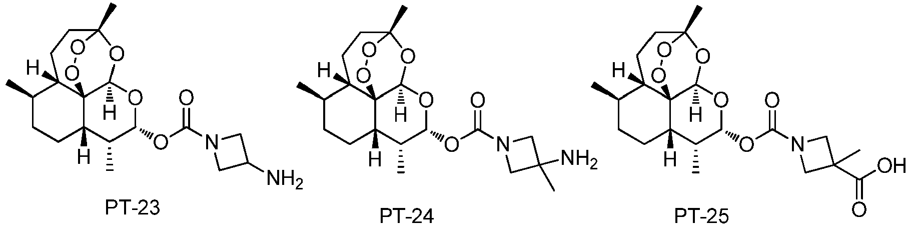 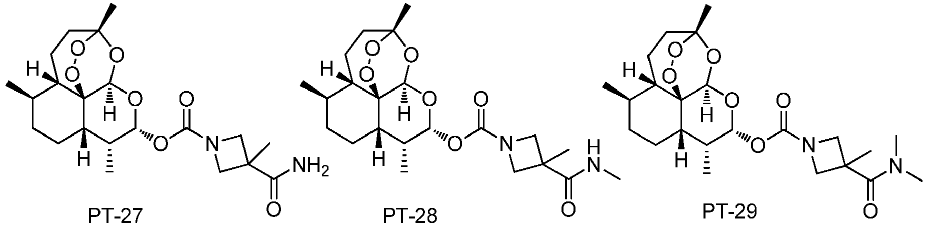 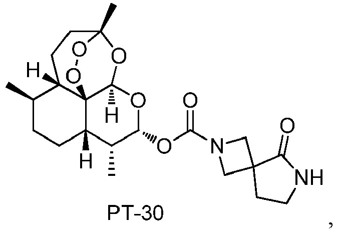 and their salts, especially their pharmaceutically acceptable salts.

These compounds are derivatives of dihydroartemisinin, being either carboxylate esters (PT-1, PT-2, PT-4) or carbamate esters (PT-13, PT-14, PT-20, PT-23 to PT-25, and PT-27 to PT-30) of dihydroartemisinin.

In a second aspect, this invention is pharmaceutical formulations for the treatment of fibrotic diseases comprising the compounds of the first aspect of this invention.

In a third aspect, this invention is the use of the compounds of the first aspect of this invention or the pharmaceutical formulations of the second aspect of this invention for the treatment of fibrotic diseases.

The compounds of any one of the formulae PT-1, PT-2, PT-4, PT-13, PT-14, PT-20, PT-23 to PT-25, and PT-27 to PT-30, and their salts, are expected to be useful in the treatment of fibrotic diseases because studies in induced pluripotent stem cell (iPSC)-derived cardiac fibroblasts activated to myofibroblasts with transforming growth factor-β (TGF-β) have shown that they give a dose-dependent decrease in the level of a fibrotic marker, because some of them have been shown to inhibit the MD2 signaling pathway, to have demonstrated antifibrotic efficacy in primary pulmonary fibroblasts, measuring the effect on acta2, col1a1, and CTGF; demonstrated antifibrotic efficacy in the CCl₄ injury mouse liver model by oral administration; and demonstrated antifibrotic efficacy in the bleomycin injury mouse skin model by topical administration; and because artesunate has been shown to be active as an antifibrotic in the bleomycin-induced pulmonary fibrosis model in rats. Moreover, because these dihydroartemisinin derivatives have a longer biological half-life than artesunate, they are expected to have greater utility than artesunate for these fibrotic diseases.

The compounds of any one of the formulae PT-1, PT-2, PT-4, PT-13, PT-14, PT-20, PT-23 to PT-25, and PT-27 to PT-30, and their salts, are expected to be able to be administered by multiple routes of administration to be an effective therapy for the fibrotic diseases mentioned in the fibrotic diseases the section entitled "Fibrotic diseases" in the Background art.

Preferred embodiments of this invention are characterized by the specification and by the features of Claims 1 to 16 of this application as filed.

### Detailed description

### Definitions

Fibrotic diseases and their treatment are described in the section entitled "Fibrotic diseases" in the Background art.

A "therapeutically effective amount" of a compound of the first aspect of this invention means that amount which, when administered for the treatment of a fibrotic disease in a subject (i.e. a human), is sufficient to effect treatment for the fibrotic disease.
"Treating" or "treatment" of a fibrotic disease in a subject includes one or more of:
(1) preventing or reducing the risk of developing a fibrotic disease, i.e., causing the clinical symptoms of the fibrotic disease not to develop in a subject who may be predisposed to a fibrotic disease but who does not yet experience or display symptoms of the fibrotic disease (i.e. prophylaxis);
(2) inhibiting a fibrotic disease, i.e., arresting or reducing the development of the fibrotic disease or its clinical symptoms; and
(3) relieving a fibrotic disease, i.e., causing regression, reversal, or amelioration of the fibrotic disease or reducing the number, frequency, duration or severity of its clinical symptoms. "Treatment" does not necessarily imply "cure" or complete treatment, e.g. treatment of all clinical symptoms of a fibrotic disease, though "treatment" may include "cure". Rather, "treatment" implies the provision of clinical benefit by the administration of the compound when compared to non-administration of the compound; and treatment may also be assessed by improvement in biological markers of the fibrotic disease being treated.
The therapeutically effective amount for a particular subject varies depending upon the health and physical condition of the subject to be treated, the nature and extent of the fibrotic disease, the assessment of the medical situation, and other relevant factors. It is expected that the therapeutically effective amount will fall in a relatively broad range that can be determined through routine trial.

"Comprising" or "containing" and their grammatical variants are words of inclusion and not of limitation and mean to specify the presence of stated components, groups, steps, and the like but not to exclude the presence or addition of other components, groups, steps, and the like. Thus "comprising" does not mean "consisting of", "consisting substantially of", or "consisting only of"; and, for example, a formulation "comprising" a compound must contain that compound but also may contain other active ingredients, prodrugs, and/or excipients. Unless the context requires otherwise, singular forms "a," "an," and "the" include plural referents.

### The compounds

The compounds of the formulae PT-1, PT-2, PT-4, PT-13, PT-14, PT-20, PT-23 to PT-25, and PT-27 to PT-30, and their salts, especially their pharmaceutically acceptable salts, may be prepared by conventional methods. For PT-1, PT-2, and PT-4, this involves esterification of dihydroartemisinin with the appropriate acid (typically activated) to give the desired carboxylate ester sidechain. For PT-13, PT-14, PT-20, PT-23 to PT-25, and PT-27 to PT-30, this involves esterification of dihydroartemisinin with 1,1-carbonyldiimidazole (CDI) followed by replacement of the imidazole ring by reaction with an appropriate amine, such as azetidin-3-ol (for PT-13), 3-methylazetidin-3-ol (for PT-14), 2,6-diazaspiro[3.4]octan-5-one (for PT-30), etc., to give the desired carbamate ester sidechain.

Where the carboxylic acid or the carbamate-forming amine to be added to the dihydroartemisinin is reactive to the reaction conditions, such as where it contains amino or hydroxy groups, then those groups will typically be protected with a protecting group, such as an acid-labile protecting group, for example *tert*-butoxycarbonyl (BOC) or similar group for an amino-containing reagent or *tert*-butyldiphenylsilyl (TBDPS) or similar group for a hydroxy-containing reagent, before the reaction forming the carboxylate or carbamate ester, and removed after the reaction.

Thus, for example, the preparation of PT-1, the 3-hydroxy-2-methylpropanoate ester of DHA, may be accomplished by the esterification of dihydroartemisinin with *O*-TBDPS-3-hydroxy-2-methylpropanoic acid in the presence of a coupling agent such as DCC (*N*,*N*'-dicyclohexylcarbodiimide) or similar compound, in the presence of an organic base such as DMAP (4-dimethylaminopyridine) in a polar aprotic solvent such as dichloromethane, followed by deprotection of the sidechain hydroxy with a reagent such as cesium fluoride in dimethylformamide, as follows:

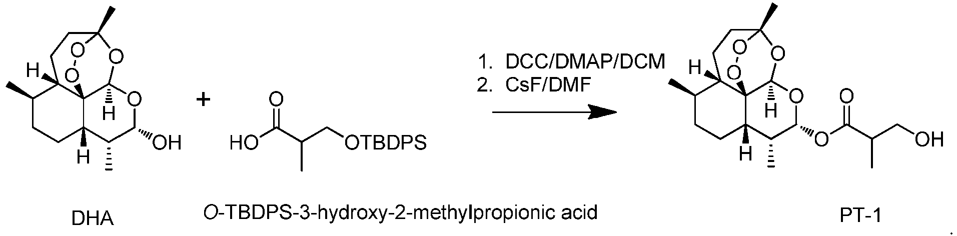

The compounds of the formulae PT-2 and PT-4 may be prepared by the same method, using appropriately protected acids, such as *O*-TBDPS-3-hydroxy-2,2-dimethylpropanoic acid (for PT-2) *O*-TBDPS-3-hydroxy-3,3-dimethylpropanoic acid (for PT-4). PT-4 may also be prepared in a single step using unprotected 3-hydroxy-3,3-dimethylpropanoic acid.

The preparation of compounds such as PT-13, PT-14, PT-20, PT-23 to PT-25, and PT-27 to PT-30 may be accomplished by esterification of dihydroartemisinin with 1,1-carbonyldiimidazole (CDI) in a polar aprotic solvent such as dichloromethane to form the 1*H*-imidazole-1-carboxylate ester, followed by replacement of the imidazole by reaction with an appropriate amine, such as azetidin-3-ol (for the preparation of PT-13), 3-methylazetidin-3-ol (for PT-14), 2,6-diazaspiro[3.4]octan-5-one (for PT-30), etc. in the presence of an organic base such as triethylamine (TEA) in a polar aprotic solvent such as dichloromethane, as follows:

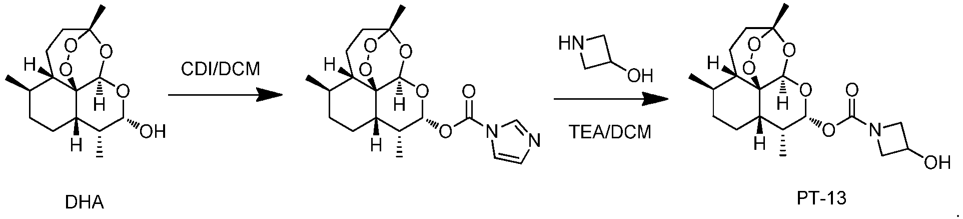

Salts (for example, pharmaceutically acceptable salts) of the compounds of any one of the formulae PT-1, PT-2, PT-4, PT-13, PT-14, PT-20, PT-23 to PT-25, and PT-27 to PT-30 are included in this invention and are useful in the methods described in this application. These salts are preferably formed with pharmaceutically acceptable acids or bases. See, for example, "Handbook of Pharmaceutically Acceptable Salts", Stahl and Wermuth, eds., Verlag Helvetica Chimica Acta, Zürich, Switzerland, for an extensive discussion of pharmaceutical salts, their selection, preparation, and use. Unless the context requires otherwise, a reference to PT-1, PT-2, PT-4, PT-13, PT-14, PT-20, PT-23 to PT-25, and PT-27 to PT-30, or to any one of those compounds, is a reference both to the compound and to its salts.

Because PT-23 and PT-24 each contain an amine group, they may form acid addition salts when the amine group reacts with inorganic acids such as hydrochloric acid, or with organic acids such as maleic acid. Typically, the compounds are treated with an excess of the acid in a protic solvent, such as water or a lower alkanol, or combination thereof, and the solvent removed sufficiently to allow crystallization of the resulting acid addition salt.

### Formulation and administration

The compounds of any one of the formulae PT-1, PT-2, PT-4, PT-13, PT-14, PT-20, PT-23 to PT-25, and PT-27 to PT-30, and their salts, may be administered by any route suitable to the subject being treated and the nature of the subject's condition. Routes of administration include administration by injection, including intravenous, intraperitoneal, intramuscular, and subcutaneous injection, by transmucosal or transdermal delivery, through topical applications, nasal spray, suppository and the like or may be administered orally. Formulations may optionally be liposomal formulations, emulsions, formulations designed to administer the drug across mucosal membranes or transdermal formulations. Suitable formulations for each of these methods of administration may be found, for example, in "Remington: The Science and Practice of Pharmacy", 20th ed., Gennaro, ed., Lippincott Williams & Wilkins, Philadelphia, Pa., U.S.A. If a compound is orally available, typical formulations will be oral, and typical dosage forms will be tablets or capsules for oral administration. Intravenous formulations may be particularly applicable for administration to acutely ill subjects, such as those subjects who may be hospitalized for treatment.

Depending on the intended mode of administration, the pharmaceutical compositions may be in the form of solid, semi-solid or liquid dosage forms, preferably in unit dosage form suitable for single administration of a precise dosage. In addition to an effective amount of the artesunate or dihydroartemisinin, the compositions may contain suitable pharmaceutically-acceptable excipients, including adjuvants which facilitate processing of the active compounds into preparations which can be used pharmaceutically. "Pharmaceutically acceptable excipient" refers to an excipient or mixture of excipients which does not interfere with the effectiveness of the biological activity of the active compound(s) and which is not toxic or otherwise undesirable to the subject to which it is administered.

For solid compositions, conventional excipients include, for example, pharmaceutical grades of mannitol, lactose, starch, magnesium stearate, sodium saccharin, talc, cellulose, glucose, sucrose, magnesium carbonate, and the like. Liquid pharmacologically administrable compositions can, for example, be prepared by dissolving, dispersing, etc., an active compound as described herein and optional pharmaceutical adjuvants in water or an aqueous excipient, such as, for example, water, saline, aqueous dextrose, and the like, to form a solution or suspension. If desired, the pharmaceutical composition to be administered may also contain minor amounts of nontoxic auxiliary excipients such as wetting or emulsifying agents, pH buffering agents and the like, for example, sodium acetate, sorbitan monolaurate, triethanolamine sodium acetate, triethanolamine oleate, etc.

For oral administration, the composition will generally take the form of a tablet or capsule; or, especially for pediatric use, it may be an aqueous or nonaqueous solution, suspension, or syrup. Tablets and capsules are preferred oral administration forms. Tablets and capsules for oral use will generally include one or more commonly used fillers such as lactose and corn starch. Lubricating agents, such as magnesium stearate, and binders, such as carbopol, are also typically added. When liquid suspensions are used, the active agent may be combined with emulsifying and suspending excipients. If desired, flavoring, coloring and/or sweetening agents may be added as well. Other optional excipients for incorporation into an oral formulation include preservatives, suspending agents, thickening agents, and the like.

Typically, a pharmaceutical composition of one of the dihydroartemisinin derivatives of the formulae PT-1 to PT-4, PT-7 to PT-20, PT-23 to PT-33, and PT-51 to PT-58, or a kit comprising a composition of the derivative, is packaged in a container with a label, or instructions, or both, indicating use of the pharmaceutical composition or kit in the treatment of a fibrotic disease.

A person of ordinary skill in the art of pharmaceutical formulation will be able to prepare suitable pharmaceutical compositions of the compounds of this invention by choosing suitable dosage forms, excipients, packaging, and the like, to achieve therapeutically effective formulations without undue experimentation and in reliance upon personal knowledge and the disclosure of this application.

A suitable (i.e., a therapeutically effective) amount of the compounds of any one of the formulae PT-1, PT-2, PT-4, PT-13, PT-14, PT-20, PT-23 to PT-25, and PT-27 to PT-30, and their salts, for systemic dosing is expected to be at least 10 mg/day and not more than 600 mg/day; for example at least 30 mg/day and not more than 400 mg/day, for an adult subject, depending on the nature, extent, and severity of the fibrotic disease and factors such as hepatic and renal function. Suitable reductions in dose toward or below the lower end of the outer range above may be made for subjects who are children, depending on such additional factors as age and body mass; and in subjects with significant hepatic or renal impairment, depending on the degree of impairment. These amounts represent an average daily dose, and not necessarily an amount given at a single dose. Dosing may be as frequent as more than once/day (where the amount, or daily dose, will be divided between the number of administrations per day), but will more typically be once/day (where the amount is given in a single administration). Optionally, particularly in cases of significant hepatic impairment, the dosing may be less frequent than once/day, such as between once/week and every other day, for example once/week, twice/week (especially with the doses at least three days apart), three times/week (especially with the doses at least two days apart), or every other day.

A person of ordinary skill in the art of the treatment of fibrotic diseases will be able to ascertain a therapeutically effective amount of the compounds of any one of the formulae PT-1, PT-2, PT-4, PT-13, PT-14, PT-20, PT-23 to PT-25, and PT-27 to PT-30, and their salts, for a particular subject and nature, extent, and severity of the fibrotic disease, to achieve a therapeutically effective amount without undue experimentation and in reliance upon personal knowledge and the disclosure of this application.

### Preparations

Abbreviations: BOC: *tert*-butoxycarbonyl; CDI: 1,1'-carbonyldiimidazole; DCC: *N,N'*-di(cyclohexyl)carbodiimide; DCM: dichloromethane; DHA: dihydroartemisinin; DIEA: *N*,*N*-diisopropylethylamine; DMF: dimethylformamide; EDCI: 1-ethyl-3-(3-dimethylaminopropyl)carbodiimide; EtOAc: ethyl acetate; HATU: Hexafluorophosphate Azabenzotriazole Tetramethyl Uronium, 1-[bis(dimethylamino)methylene]-1*H*-1,2,3-triazolo[4,5-b]pyridinium 3-oxide hexafluorophosphate; LCMS: liquid chromatography-mass spectrometry; TBDPSCl: *tert*-butyldiphenylsilyl chloride; TEA: triethylamine; TEMPO: (2,2,6,6-tetramethylpiperidin-1-yl)oxyl; TFA: trifluoroacetic acid; THF: tetrahydrofuran; TLC: thin-layer chromatography.

### Preparation 1: Preparation of PT-1, DHA 3-hydroxy-2-methylpropionate

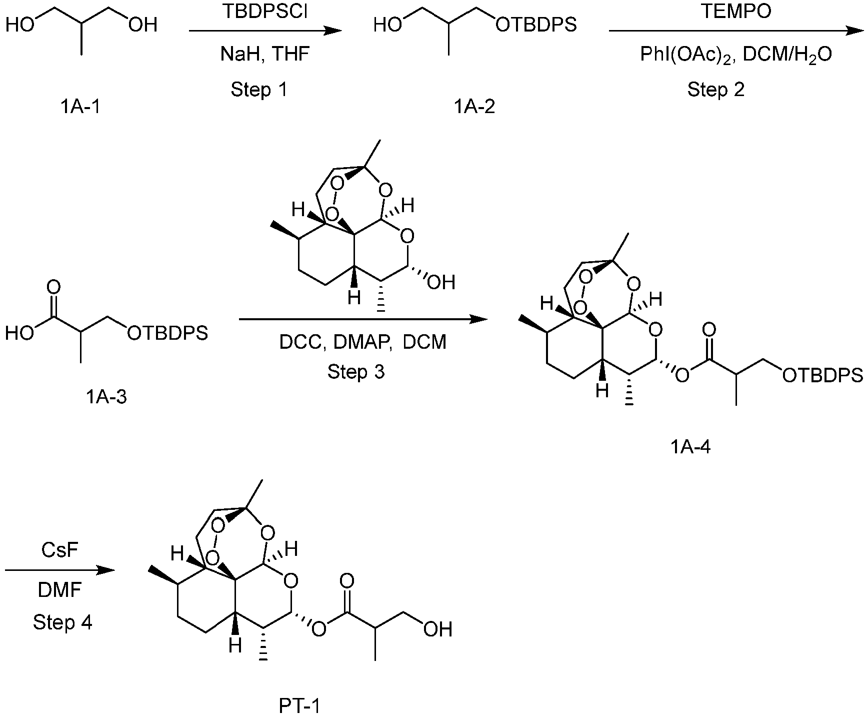

Step 1: 3-((*tert*-Butyldiphenylsilyl)oxy)-2-methylpropan-1-ol (1A-2) To a solution of 2-methylpropane-1,3-diol (6.00 g, 66.6 mmol, 5.90 mL, 1.0 eq) in THF (90 mL) was added NaH (2.90 g, 73.2 mmol, 60% purity, 1.1 eq) in portions at 0 °C, then the mixture was stirred at 0 °C for 10 min. TBDPSCl (20.1 g, 73.2 mmol, 18.7 mL, 1.1 eq) was added portionwise into the reaction, which was stirred at 20 °C for 12 h. LCMS indicated that the starting material was completely consumed, and the desired mass was detected. The reaction mixture was allowed to cool to 0 °C, poured into saturated aqueous NH₄Cl (100 mL) and extracted with EtOAc (100 mL × 3). The combined organic phase was washed with brine (100 mL), dried over Na₂SO₄, filtered and concentrated under vacuum to give a residue which was purified by flash silica gel chromatography using a gradient of EtOAc/petroleum ether from 0/1 to 1/9. 3-((*tert*-Butyldiphenylsilyl)oxy)-2-methylpropan-1-ol (13.0 g, 39.6 mmol) was obtained as a colorless oil. ¹H NMR (400 MHz, CDCl₃) δ 7.70 (dd, *J =* 1.5, 7.8 Hz, 4H), 7.52 - 7.37 (m, 6H), 3.78 - 3.55 (m, 4H), 2.56 (br s, 1H), 2.05 - 1.93 (m, 1H), 1.08 (s, 9H), 0.85 (d, *J* = 6.9 Hz, 3H).

### Step 2: 3-((tert-Butyldiphenylsilyl)oxy)-2-methylpropanoic acid (1A-3)

To a solution of 3-((*tert*-butyldiphenylsilyl)oxy)-2-methylpropan-1-ol (5.50 g, 16.7 mmol, 1.0 eq) in DCM (330 mL) was added TEMPO (527 mg, 3.40 mmol, 0.2 eq), H₂O (15.1 g, 837 mmol, 15.1 mL, 50.0 eq) and PhI(OAc)₂ (13.5 g, 41.9 mmol, 2.5 eq). The mixture was stirred at 20 °C for 12 h. LCMS indicated that the starting material was completely consumed, and the desired mass was detected. The reaction mixture was poured into saturated aqueous solution of Na₂S₂O₃ (60 mL) and extracted with DCM (30 mL × 3). The combined organic layers were washed with brine (60 mL), dried over Na₂SO₄, filtered, and concentrated under vacuum to give a residue which was purified by flash silica gel chromatography using a gradient of EtOAc/petroleum ether from 0/1 to 3/7. 3-(*(tert*-Butyldiphenylsilyl)oxy)-2-methylpropanoic acid (2.50 g, 7.30 mmol) was obtained as a white solid. ¹H NMR (400 MHz, CDCl₃) δ 7.72 - 7.65 (m, 4H), 7.51 - 7.36 (m, 6H), 3.87 - 3.73 (m, 2H), 2.74 (dt, *J* = 5.6, 7.1 Hz, 1H), 1.19 (d, *J =* 7.0 Hz, 3H), 1.05 (s, 9H).

### Step 3: DHA 3-((tert-butyldiphenylsilyl)oxy)-2-methylpropanoate (1A-4)

To a solution of DHA (2.20 g, 7.60 mmol, 2.0 eq) in DCM (50 mL) was added 3-((*tert*-butyldiphenylsilyl)oxy)-2-methylpropanoic acid (1.30 g, 3.8 mmol, 1.0 eq), DCC (1.60 g, 7.60 mmol, 1.5 mL, 2.0 eq) and DMAP (46.4 mg, 379 µmol, 0.1 eq), and the resulting mixture was stirred at 20 °C for 12 h. LCMS indicated that the starting material was completely consumed, and the desired mass was detected. The reaction mixture was filtered and concentrated under vacuum to give a residue which was purified by flash silica gel chromatography using a gradient of EtOAc/petroleum ether from 0/1 to 1/9. DHA 3-((*tert*-butyldiphenylsilyl) oxy)-2-methylpropanoate (1.4 g, crude) was obtained as a white solid. ¹H NMR (400 MHz, CDCl₃) δ 7.75 - 7.60 (m, 4H), 7.48 - 7.34 (m, 6H), 5.88 - 5.77 (m, 1H), 5.45 (s, 1H), 3.96 - 3.71 (m, 2H), 2.84 - 2.71 (m, 1H), 2.65 - 2.53 (m, 1H), 2.38 (dt, *J =* 3.9, 14.0 Hz, 1H), 2.08 - 1.99 (m, 1H), 1.94 - 1.85 (m, 1H), 1.82 - 1.68 (m, 2H), 1.67 - 1.46 (m, 3H), 1.45 - 1.38 (m, 4H), 1.37 - 1.14 (m, 6H), 1.04 (s, 9H), 0.97 (d, *J =* 6.0 Hz, 3H), 0.87 - 0.83 (m, 3H).

### Step 4: DHA 3-hydroxy-2-methylpropanoate (PT-1)

To a solution of DHA 3-((*tert*-butyldiphenylsilyl)oxy)-2-methylpropanoate (1.30 g, 2.10 mmol, 1.0 eq) in DMF (26 mL) was added CsF (973 mg, 6.40 mmol, 237 µL, 3.0 eq). The mixture was stirred at 20 °C for 13 h. LCMS indicated that the starting material was completely consumed. The reaction mixture was poured into H₂O (50 mL) and extracted with EtOAc (30 mL × 3). The combined organic layers were dried over Na₂SO₄, filtered and concentrated under vacuum to give a residue which was purified by flash silica gel chromatography using a gradient of EtOAc/petroleum ether from 0/1 to 3/7. DHA 3-hydroxy-2-methylpropanoate (60.0 mg) was obtained as a white solid. M + Na = 393.2 (LCMS); ¹H NMR (400 MHz, CDCl₃) δ 5.87 - 5.78 (m, 1H), 5.50 - 5.42 (m, 1H), 3.86 - 3.68 (m, 2H), 2.86 - 2.72 (m, 1H), 2.67 - 2.54 (m, 1H), 2.39 (dt, *J* = 3.9, 13.9 Hz, 1H), 2.09 - 2.01 (m, 1H), 1.90 (ddd, *J* = 3.3, 6.4, 13.6 Hz, 1H), 1.84 - 1.71 (m, 3H), 1.64 (td, *J* = 4.5, 13.8 Hz, 1H), 1.52 - 1.42 (m, 4H), 1.39 - 1.20 (m, 6H), 1.10 - 0.96 (m, 4H), 0.91 - 0.85 (m, 3H).

### Preparation 2: Preparation of PT-2, DHA 3-hydroxy-2,2-dimethylpropanoate

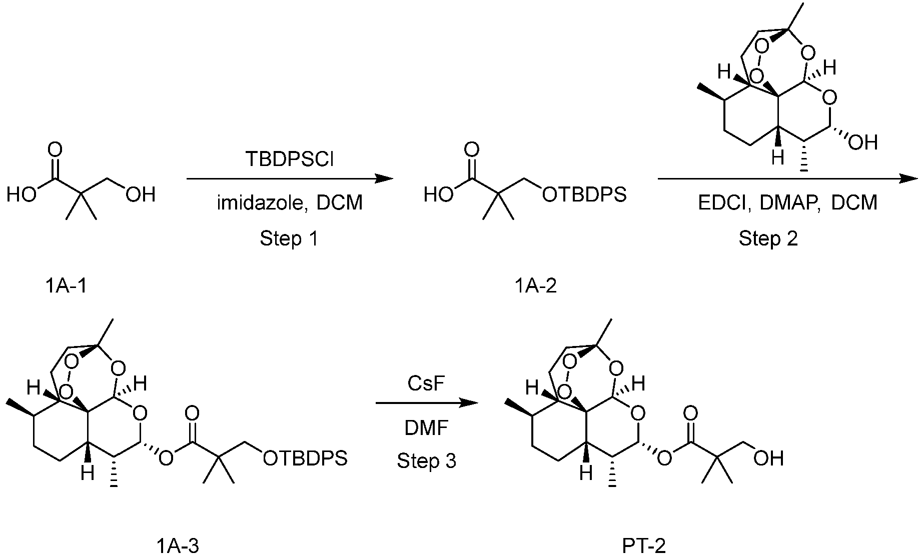

### Step 1: 3-((tert-Butyldiphenylsilyl)oxy)-2,2-dimethylpropanoic acid (1A-2)

To a solution of 3-hydroxy-2,2-dimethylpropanoic acid (2.00 g, 16.9 mmol, 1.0 eq) and imidazole (1.27 g, 18.6 mmol, 1.1 eq) in DCM (50 mL) was added TBDPSCl (5.12 g, 18.6 mmol, 4.77 mL, 1.1 eq) at 0°C, and the resulting mixture was stirred at 20 °C for 1 h. TLC (EtOAc/petroleum ether 1/5, R*_{f}*= 0.69) indicated that the starting material was completely consumed. The reaction mixture was poured into HCl (1 M aqueous, 30 mL) and extracted with DCM (30 mL × 3). The combined organic layers were washed with brine (20 mL × 3), dried over Na₂SO₄, filtered and concentrated under vacuum to give a residue which was purified by flash silica gel chromatography using a gradient of EtOAc/petroleum ether from 0/1 to 1/10. 3-((*tert*-Butyldiphenylsilyl)oxy)-2,2-dimethylpropanoic acid (5.00 g, 14.0 mmol, 83% yield) was obtained as a white solid. ¹H NMR (400 MHz, CDCl₃) δ 7.67 (dd, *J =* 1.4, 7.8 Hz, 4H), 7.45 - 7.36 (m, 6H), 3.66 (s, 2H), 1.23 (s, 6H), 1.06 (s, 9H).

### Step 2: DHA 3-((tert-butyldiphenylsilyl)oxy)-2,2-dimethyl propanoate (1A-3)

To a solution of 3-((*tert*-butyldiphenylsilyl)oxy)-2,2-dimethylpropanoic acid (2.51 g, 7.03 mmol, 2.0 eq) in DCM (60 mL) was added (3*R*,5a*S*,6*R*,8a*S*,9*R*,10*S*,12*R*,12a*R*)-3,6,9-trimethyldecahydro-12*H*-3,12-epoxy[1,2]dioxepino[4,3-*i*]isochromen-10-ol (1.00 g, 3.52 mmol, 1.0 eq) and DMAP (430 mg, 3.52 mmol, 1.0 eq), EDCI (1.35 g, 7.03 mmol, 2.0 eq), and the resulting mixture was stirred at 20 °C for 12 h. TLC (EtOAc/petroleum ether = 1/5, R*_{f}*= 0.64) indicated that the starting material was completely consumed. The reaction mixture was concentrated under vacuum to give a residue which was purified by flash silica gel chromatography using a gradient of EtOAc/petroleum ether from 0/1 to 1/0. DHA 3-((tert-butyldiphenylsilyl)oxy)-2,2-dimethyl propanoate (1.90 g, 3.05 mmol, 87% yield) was obtained as a white solid. ¹H NMR (400 MHz, CDCl₃) δ 7.78 - 7.61 (m, 5H), 7.47 - 7.34 (m, 7H), 5.79 (d, *J* = 9.9 Hz, 1H), 5.44 (s, 1H), 3.75 (d, *J* = 9.5 Hz, 1H), 3.62 (d, *J* = 9.5 Hz, 1H), 2.67 - 2.53 (m, 1H), 2.37 (dt, *J =* 3.9, 14.0 Hz, 1H), 2.08 - 1.98 (m, 1H), 1.89 (quind, *J* = 3.3, 13.7 Hz, 1H), 1.81 - 1.69 (m, 2H), 1.66 - 1.58 (m, 1H), 1.54 - 1.16 (m, 10H), 1.10 - 1.02 (m, 11H), 0.97 (d, *J =* 6.0 Hz, 3H), 0.81 (d, *J* = 7.3 Hz, 3H).

### Step 3: DHA 3-hydroxy-2,2-dimethylpropanoate (PT-2)

To a solution of DHA 3-((*tert*-butyldiphenylsilyl)oxy)-2,2-dimethyl propanoate (500 mg, 803 µmol, 1.0 eq) in DMF (20 mL) was added CsF (366 mg, 2.41 mmol, 88.9 µL, 3.0 eq) at 20 °C, the resulting mixture was stirred at 20 °C for 12 h. TLC (EtOAc/petroleum ether = 1/1, R*_{f}* = 0.24) indicated that some of the starting material was remained and the desired spot was detected. The reaction mixture was poured into H₂O (30 mL) and extracted with EtOAc (20 mL × 5). The combined organic layers were washed with brine (10 mL × 3), dried over Na₂SO₄, filtered and concentrated under vacuum to give a residue which was purified by flash silica gel chromatography using a gradient of EtOAc/petroleum ether from 0/1 to 1/1. DHA 3-hydroxy-2,2-dimethylpropanoate (52.0 mg, 135 µmol, 6% yield) was obtained as a white solid. M + Na = 407.2; ¹H NMR (400 MHz, CDCl₃) δ 5.78 (d, *J* = 9.9 Hz, 1H), 5.44 (s, 1H), 3.68 (d, *J* = 11.3 Hz, 1H), 3.53 (d, *J =* 11.3 Hz, 1H), 2.67 - 2.54 (m, 1H), 2.38 (dt, *J* = 3.9*,* 14.0 Hz, 1H), 2.09 - 1.85 (m, 3H), 1.83 - 1.58 (m, 4H), 1.56 - 1.15 (m, 11H), 1.20 - 0.94 (m, 5H), 0.87 (d, *J =* 7.1 Hz, 3H).

### Preparation 3: Preparation of PT-4, DHA 3-hydroxy-3-methylbutanoate

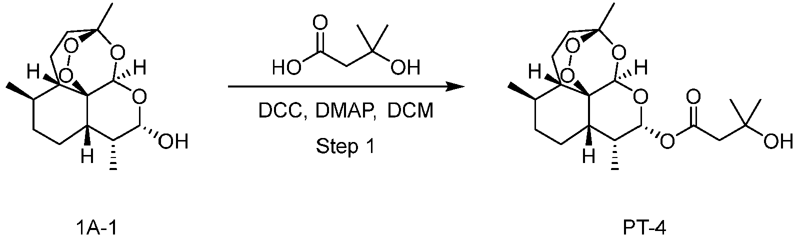

To a solution of 3-hydroxy-3-methylbutanoic acid (500 mg, 4.23 mmol, 1.0 eq) in DCM (30 mL) was added DHA (2.41 g, 8.47 mmol, 2.0 eq) and DCC (1.75 g, 8.47 mmol, 1.71 mL, 2.0 eq), DMAP (51.7 mg, 423 µmol, 0.1 eq), the resulting mixture was stirred at 20 °C for 12 h. LCMS indicated that the starting material was completely consumed and the desired mass was detected. The reaction mixture was filtered, and the filtrate was concentrated under vacuum to give a residue which was purified by flash silica gel chromatography using a gradient of EtOAc/petroleum ether from 0/1 to 1/0. Then was purified by preparative HPLC (Phenomenex Luna C18 column (75 × 30 mm, 3 µm); flow rate: 25 mL/min; gradient: 45% - 70% B over 8 min; mobile phase A: 0.04% aqueous HCl, mobile phase B: acetonitrile). DHA 3-hydroxy-3-methylbutanoate (242 mg, 623 µmol, 15% yield) was obtained as a white solid. M + Na = 407.2 (LCMS); ¹H NMR (400 MHz, CDCl₃) δ 5.85 (d, *J =* 9.9 Hz, 1H), 5.47 (s, 1H), 2.65 - 2.52 (m, 3H), 2.40 (ddd, *J =* 3.9, 13.5, 14.5 Hz, 1H), 2.10 - 2.01 (m, 1H), 1.97 - 1.88 (m, 1H), 1.86 - 1.71 (m, 2H), 1.66 (td, *J* = 4.5, 13.7 Hz, 1H), 1.57 - 1.44 (m, 5H), 1.45 - 1.27 (m, 9H), 1.04 - 0.94 (m, 4H), 0.88 (d, *J* = 7.1 Hz, 3H).

### Preparation 4: Preparation of PT-13, DHA 3-hydroxyazetidine-1-carboxylate

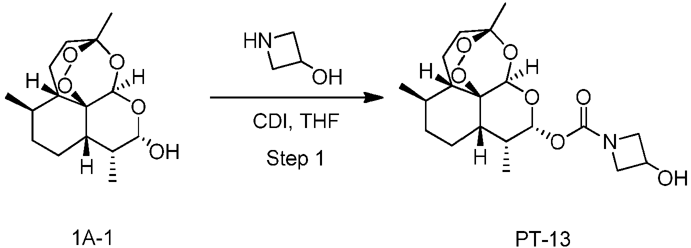

To a solution of CDI (684 mg, 4.22 mmol, 1.2 eq) in DCM (100 mL) was added DHA (1.00 g, 3.52 mmol, 1.0 eq). The reaction mixture was stirred at 20 °C for 10 min, then azetidin-3-ol (385 mg, 3.52 mmol, 1.0 eq, HCl salt) and TEA (427 mg, 4.22 mmol, 587 µL, 1.2 eq) were added. The resulting mixture was stirred at 20 °C for 12 h. LCMS indicated that the starting material was completely consumed and the desired mass was detected. The reaction mixture was concentrated under vacuum to give a residue which was purified by flash silica gel chromatography using a gradient of EtOAc/petroleum ether from 0/1 to 1/0. DHA 3-hydroxyazetidine-1-carboxylate (270 mg, 698 µmol, 20% yield) was obtained as a white solid. M - H⁻ = 382.2 (LCMS); ¹H NMR (400 MHz, CDCl₃) δ 5.66 (d, *J =* 9.8 Hz, 1H), 5.45 (s, 1H), 4.65 (br d, *J* = 4.6 Hz, 1H), 4.47 - 4.18 (m, 2H), 4.11 - 3.81 (m, 2H), 2.60 - 2.47 (m, 1H), 2.37 (dt, *J =* 3.9, 14.0 Hz, 1H), 2.22 (br s, 1H), 2.10 - 1.94 (m, 1H), 1.94 - 1.85 (m, 1H), 1.82 - 1.68 (m, 2H), 1.66 - 1.60 (m, 1H), 1.59 - 1.40 (m, 4H), 1.40 - 1.22 (m, 3H), 1.07 - 0.93 (m, 4H), 0.86 (d, J = 7.0 Hz, 3H).

### Preparation 5: Preparation of PT-14, DHA 3-hydroxyazetidine-1-carboxylate, (3R,5aS,6R,8aS,9R,12S,12aR)-decahydro-3,6,9-trimethyl-3,12-epoxy-12H-pyrano[4,3-j]1,2-benzodioxepin-10-yl 3-hydroxyazetidine-1-carboxylate

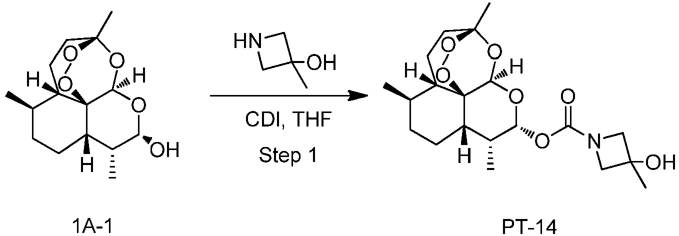

To a solution of CDI (6.84 g, 42.2 mmol, 1.2 eq) in DCM (400 mL) was added DHA (10.0 g, 35.2 mmol, 1.0 eq) and the reaction mixture was stirred at 25 °C for 3 h. Then 3-methylazetidin-3-ol (4.35 g, 35.2 mmol, 1.0 eq, HCl salt) and TEA (4.27 g, 42.2 mmol, 5.87 mL, 1.2 eq) were added, the resulting mixture was stirred at 25 °C for 12 h. LCMS indicated that the starting material was completely consumed and the desired mass was detected. The reaction mixture was concentrated under vacuum to give a residue which was purified by flash silica gel chromatography using a gradient of EtOAc/petroleum ether from 0/1 to 1/1. DHA 3-hydroxy-3-methylazetidine-1-carboxylate (5.14 g, 12.91 mmol, 36.7% yield) was obtained as a white solid. M - H⁻ = 396.2 (LCMS); ¹H NMR (400 MHz, CDCl₃) δ 5.67 (d, *J* = 9.8 Hz, 1H), 5.45 (s, 1H), 4.19 - 3.81 (m, 4H), 2.55 (ddd, *J* = 4.6, 7.2, 9.9 Hz, 1H), 2.38 (dt, *J* = 3.9, 14.0 Hz, 1H), 2.09 - 1.99 (m, 2H), 1.90 (ddd, *J* = 3.4*,* 6.4, 13.9 Hz, 1H), 1.81 - 1.69 (m, 2H), 1.66 - 1.59 (m, 1H), 1.54 (s, 3H), 1.53 - 1.23 (m, 7H), 1.06 - 0.93 (m, 4H), 0.87 (d, *J =* 7.1 Hz, 3H).

PT-20, DHA (2-hydroxyethyl)(methyl)carbamate, was similarly prepared using 2-methylamino)ethan-1-ol instead of 3-methylazetidin-3-ol, giving DHA (2-hydroxyethyl)(methyl)carbamate (640 mg, 1.66 mmol, 50% yield) as a white solid after purification. M + H⁺ = 386.3 (LCMS); ¹H NMR (400 MHz, CDCl₃) δ 5.62 (br t, *J* = 10.9 Hz, 1H), 5.37 (br d, *J* = 7.3 Hz, 1H), 3.76 - 3.65 (m, 2H), 3.54 - 3.29 (m, 2H) , 2.95 (br d, *J =* 15.4 Hz, 3H), 2.59 - 2.15 (m, 3H), 2.03 - 1.91 (m, 1H), 1.88 - 1.77 (m, 1H) , 1.75 - 1.61 (m, 2H), 1.60 - 1.49 (m, 1H), 1.48 - 1.30 (m, 5H), 1.29 - 1.15 (m, 2H), 0.99 - 0.86 (m, 4H), 0.81 (d, *J* = 7.1 Hz, 3H).

PT-24, DHA 3-amino-3-methylazetidine-1-carboxylate, was similarly prepared using 3-methylazetidin-3-amine instead of 3-methylazetidin-3-ol, giving DHA 3-amino-3-methylazetidine-1-carboxylate (300 mg, 756 µmol, 43% yield) as a white solid after purification. M + H⁺ = 397.2 (LCMS); ¹H NMR (400 MHz, DMSO-*d₆*) δ 5.53 (s, 1H), 5.49 (br d, *J =* 9.5 Hz, 1H), 3.80 - 3.60 (m, 4H), 2.31 - 2.13 (m, 4H), 2.04 - 1.96 (m, 1H), 1.87 - 1.76 (m, 1H), 1.67 - 1.51 (m, 3H), 1.49 - 1.39 (m, 2H), 1.35 (br d, *J* = 4.5 Hz, 1H), 1.29 (s, 6H), 1.17 (dt, *J* = 6.6, 11.3 Hz, 1H), 1.00 - 0.92 (m, 1H), 0.89 (d, *J=* 6.3 Hz, 3H), 0.83 - 0.74 (m, 3H).

PT-25, DHA 3-carboxy-3-methylazetidine-1-carboxylate, was similarly prepared using 3-methylazetidin-3-carboxylic acid instead of 3-methylazetidin-3-ol, giving DHA 3-carboxy-3-methylazetidine-1-carboxylate (200 mg, 467 µmol, 9% yield) as a white solid after purification. M + H⁺ = 426.2 (LCMS); ¹H NMR (400 MHz, DMSO-*d₆*) δ 13.13 - 12.66 (m, 1H), 5.54 (s, 1H), 5.50 (d, *J =* 9.8 Hz, 1H), 4.26 - 4.06 (m, 2H), 3.86 - 3.66 (m, 2H), 2.28 (ddd, *J =* 4.4, 6.9, 9.8 Hz, 1H), 2.19 (dt, *J =* 3.8, 14.0 Hz, 1H), 2.04 - 1.96 (m, 1H), 1.85 - 1.77 (m, 1H), 1.67 - 1.58 (m, 2H), 1.57 - 1.51 (m, 1H), 1.48 (br s, 1H), 1.46 (br s, 3H), 1.43 - 1.38 (m, 1H), 1.38 - 1.32 (m, 1H), 1.30 (s, 3H), 1.17 (dt, *J* = 6.7, 11.3 Hz, 1H), 1.01 - 0.92 (m, 1H), 0.89 (d, *J* = 6.3 Hz, 3H), 0.79 (d, *J* = 7.0 Hz, 3H).

### Preparation 6: Preparation of PT-28, DHA 3-methyl-3-(methylcarbamoyl)azetidine-1-carboxylate

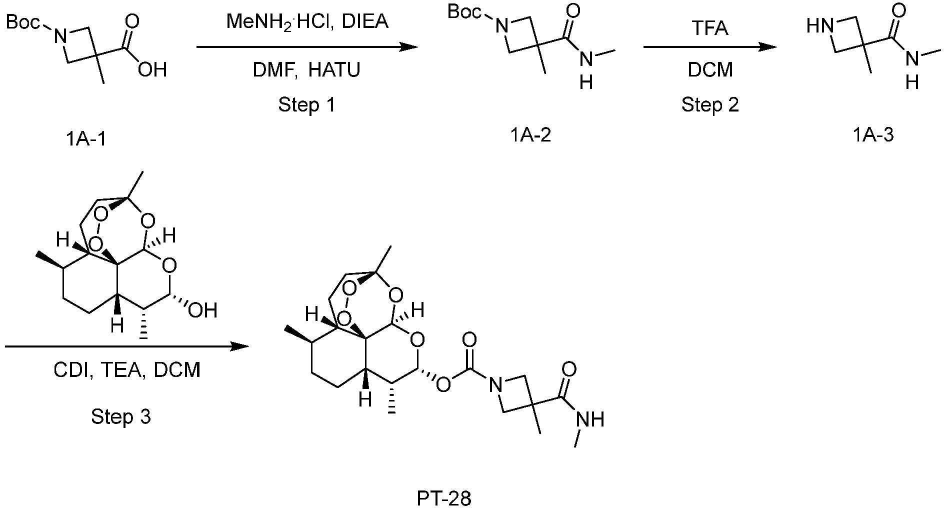

### Step 1: tert-Butyl 3-methyl-3-(methylcarbamoyl)azetidine-1-carboxylate (1A-2)

To a solution of 1-(*tert*-butoxycarbonyl)-3-methylazetidine-3-carboxylic acid (500 mg, 2.32 mmol, 1.0 eq), methanamine (235 mg, 3.48 mmol, 1.5 eq, HCl salt) and DIEA (1.20 g, 9.29 mmol, 1.60 mL, 4.0 eq) in DMF (5.0 mL) was added HATU (1.32 g, 3.48 mmol, 1.5 eq). The mixture was stirred at 30 °C for 12 h. LCMS indicated that the starting material was completely consumed and the desired mass was detected. The reaction mixture was poured into HCl (1 M aqueous, 10 mL) at 0 °C and extracted with EtOAc (20 mL × 3). The combined organic layers were dried over Na₂SO₄, filtered and concentrated under vacuum to give a residue which was purified by flash silica gel chromatography using a gradient of EtOAc/petroleum ether from 0/1 to 1/0. tert-Butyl 3-methyl-3-(methylcarbamoyl)azetidine-1-carboxylate (500 mg, 2.19 mmol, 94% yield) was obtained as a yellow oil. M + H⁺ =229.2 (LCMS); ¹H NMR (400 MHz, CDCl₃) δ 4.11 (d, *J =* 8.5 Hz, 2H), 3.59 (d, *J =* 8.4 Hz, 2H), 2.89 - 2.82 (m, 3H), 2.78 (d, *J =* 4.8 Hz, 3H), 1.41 - 1.34 (m, 9H).

### Step 2: N,3-dimethylazetidine-3-carboxamide (1A-3)

A mixture of *t*er*t*-butyl 3-methyl-3-(methylcarbamoyl)azetidine-1-carboxylate (500 mg, 2.19 mmol, 1.0 eq) in DCM (3.0 mL) and TFA (1.0 mL) was stirred at 25°C for 1 h. LCMS indicated that the starting material was completely consumed and the desired mass was detected. The reaction mixture was concentrated under vacuum to give *N,*3-dimethylazetidine-3-carboxamide (530 mg, crude, TFA salt) as a yellow oil. M + H⁺ = 129.1 (LCMS).

### Step 3: DHA 3-methyl-3-(methylcarbamoyl)azetidine-1-carboxylate (PT-28)

To a solution of CDI (328 mg, 2.03 mmol, 1.2 eq) in DCM (6.0 mL) was added DHA (480 mg, 1.69 mmol, 1.0 eq). The mixture was stirred at 30 °C for 0.5 h. Then *N*,3-dimethylazetidine-3-carboxamide (523 mg, 2.16 mmol, 1.2 eq, TFA salt) and TEA (256 mg, 2.53 mmol, 352 µL, 1.5 eq) in DCM (2.0 mL) was added to the above solution. The mixture was stirred at 30 °C for 0.5 h. LCMS indicated that the starting material was completely consumed and the desired mass was detected. The reaction mixture was concentrated under vacuum to give a residue which was purified by flash silica gel chromatography using a gradient of EtOAc/petroleum ether from 0/1 to 1/0, then was further purified by preparative HPLC (Phenomenex Luna C18 column (78 × 30 mm, 3 µm); flow rate: 25 mL/min; gradient: 45% - 75% B over 9 min; mobile phase A: 10 mm aqueous NH₄HCO₃, mobile phase B: acetonitrile). DHA 3-methyl-3-(methylcarbamoyl)azetidine-1-carboxylate (180 mg, 400 µmol, 23% yield) was obtained as a white solid. M + H⁺ = 439.2 (LCMS); ¹H NMR (400 MHz, CDCl₃) δ 5.67 (d, *J =* 9.8 Hz, 1H), 5.46 (s, 1H), 4.46 - 4.17 (m, 2H), 3.93 - 3.68 (m, 2H), 2.88 (d, J = 4.8 Hz, 3H), 2.63 - 2.49 (m, 1H), 2.39 (dt, *J =* 3.9, 14.0 Hz, 1H), 2.10 - 1.99 (m, 1H), 1.96 - 1.85 (m, 1H), 1.84 - 1.70 (m, 2H), 1.64 (s, 1H), 1.62 - 1.58 (m, 1H), 1.57 (s, 3H), 1.45 (s, 3H), 1.44 - 1.39 (m, 1H), 1.38 - 1.24 (m, 2H), 1.08 - 1.00 (m, 1H), 0.98 (d, *J =* 6.0 Hz, 3H), 0.88 (d, *J=* 7.0 Hz, 3H).

PT-27, DHA 3-methyl-3-carbamoylazetidine-1-carboxylate, was similarly prepared using 3-methylazetidine-3-carboxamide [prepared from 1-(*t*er*t*-butoxycarbonyl)-3-methylazetidine-3-carboxylic acid by reaction with ammonium acetate/DIEA/HATU in DMF and subsequent deprotection] instead of *N,*3-dimethylazetidine-3-carboxamide, giving DHA 3-methyl-3-carbamoylazetidine-1-carboxylate (190 mg, 447 µmol, 18% yield) as a white solid after purification. M + H⁺ = 425.2 (LCMS); ¹H NMR (400 MHz, DMSO-*d₆*) δ 7.43 (br s, 1H), 7.12 (br s, 1H), 5.53 (s, 1H), 5.49 (d, *J =* 9.6 Hz, 1H), 4.23 - 4.03 (m, 2H), 3.76 - 3.55 (m, 2H), 2.31 - 2.22 (m, 1H), 2.17 (br dd, *J* = 3.6, 13.8 Hz, 1H), 2.04 - 1.96 (m, 1H), 1.86 - 1.76 (m, 1H), 1.67 - 1.51 (m, 3H), 1.46 (br s, 4H), 1.42 - 1.32 (m, 2H), 1.30 (s, 3H), 1.21 - 1.13 (m, 1H), 0.99 - 0.92 (m, 1H), 0.89 (br d, *J =* 6.4 Hz, 3H), 0.82 - 0.76 (m, 3H).

PT-29, DHA 3-methyl-3-(dimethylcarbamoyl)azetidine-1-carboxylate, was similarly prepared using 3-methylazetidine-3-dimethylcarboxamide [prepared from 1-(*t*er*t*-butoxycarbonyl)-3-methylazetidine-3-carboxylic acid by reaction with dimethylamine/DIEA/HATU in DMF and subsequent deprotection] instead of N,3-dimethylazetidine-3-carboxamide, giving DHA 3-methyl-3-(dimethylcarbamoyl)azetidine-1-carboxylate (110 mg, 240 µmol, 15% yield) as a white solid after purification. M + H⁺ = 453.3 (LCMS); ¹H NMR (400 MHz, CDCl₃) δ 5.66 (br d, *J =* 8.8 Hz, 1H), 5.45 (s, 1H), 4.59 - 4.24 (m, 2H), 3.97 - 3.67 (m, 2H), 2.97 (s, 3H), 2.88 - 2.82 (m, 3H), 2.55 (ddd, *J* = 4.6, 6.9, 9.9 Hz, 1H), 2.38 (dt, *J =* 3.9, 14.0 Hz, 1H), 2.05 (td, *J* = 3.5, 14.6 Hz, 1H), 1.95 - 1.84 (m, 1H), 1.81 - 1.69 (m, 2H), 1.66 - 1.62 (m, 1H), 1.60 (s, 3H), 1.59 - 1.48 (m, 1H), 1.45 (s, 3H), 1.43 (s, 1H), 1.38 - 1.24 (m, 2H), 1.07 - 0.94 (m, 4H), 0.92 - 0.82 (m, 3H).

PT-23, DHA 3-aminoazetidine-1-carboxylate, was prepared by a similar method, but required protection and deprotection of the amine. Benzyl 3-aminoazetidine-1-carboxylate in DCM was converted to benzyl 3-(2,2,2-trifluoroacetamido)azetidine-1-carboxylate by reaction with 1.5 equivalents of trifluoroacetic anhydride, 3.0 equivalents of TEA, and 0.1 equivalents of DMAP at 25 °C for 1 h, then worked up by acidification with 1M hydrochloric acid, extraction into ethyl acetate, and the extracts concentrated and purified by flash chromatography. The benzyl 3-(2,2,2-trifluoroacetamido)azetidine-1-carboxylate was debenzylated by reduction with hydrogen gas (1 atm) and 10% Pd/C in ethyl acetate, filtered, and concentrated under vacuum to give *N*-(azetidin-3-yl)-2,2,2-trifluoroacetamide. This was reacted with DHA/CDI/TEA in DCM in the same manner as for PT-28, concentrated under vacuum, and purified by flash silica gel chromatography to give DHA 3-(2,2,2-trifluoroacetamido)azetidine-1-carboxylate as a white solid. This was reacted with 1.5 equivalents of sodium hydroxide in ethanol/water, filtered, and purified by preparative HPLC to give DHA 3-aminoazetidine-1-carboxylate (30.0 mg, 70.6 µmol, 6% yield, HCl salt) as a white solid. M + H⁺ = 383.2 (LCMS); ¹H NMR (400 MHz, DMSO) δ 9.16 - 8.49 (m, 3H), 6.26 (d, *J* = 3.9 Hz, 1H), 5.43 (s, 1H), 5.01 - 4.95 (m, 1H), 4.18 - 4.04 (m, 4H), 2.38 - 2.26 (m, 1H), 2.18 (dt, *J* = 3.8, 13.9 Hz, 1H), 2.05 - 1.74 (m, 3H), 1.70 - 1.52 (m, 2H), 1.45 - 1.30 (m, 3H), 1.30 - 1.21 (m, 3H), 1.18 - 1.09 (m, 1H), 0.98 - 0.74 (m, 7H).

### Preparation 7: Preparation of PT-30, DHA 5-oxo-2,6-diazaspiro[3.4]octane-2-carboxylate, (3R,5aS,6R,8aS,9R,12S,12aR)-decahydro-3,6,9-trimethyl-3,12-epoxy-12H-pyrano[4,3-j]-1,2-benzodioxepin-10-yl 5-oxo-2,6-diazaspiro[3.4]octane-2-carboxylate

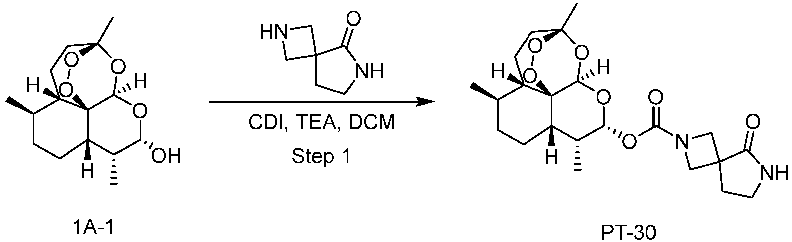

2,6-diazaspiro[3.4]octan-5-one was prepared by a five-step process from methyl *N*-BOC-azetidine-3-carboxylate. Methyl N-BOC-azetidine-3-carboxylate was reacted with 1.1 equivalents each of allyl bromide and lithium hexamethyldisilazane in THF at 25 °C for 2 h to give methyl *N*-BOC-3-allylazetidine-3-carboxylate; and this was oxidized with 2.5 equivalents of sodium periodate and 0.2 equivalents of potassium osmate in 1:1 THF/water at 25 °C for 2 h to give methyl *N*-BOC-3-(2-oxoethyl)azetidine-3-carboxylate. This was reacted with 1.5 equivalents of hydroxylamine hydrochloride in methanol at 25 - 80 °C over 2 h to give methyl *N*-BOC-3-(2-(hydroxylimino)oxoethyl)azetidine-3-carboxylate; which was cyclized with hydrogen over 10% Ru/SiO₂ using flow chemistry to give *N*²-BOC-2,6-diazaspiro[3.4]octan-5-one. Finally, the *N*²-BOC-2,6-diazaspiro[3.4]octan-5-one was deprotected with 1.2 equivalents of 4-toluenesulfonic acid in acetonitrile at 80 °C for 16 h to give 2,6-diazaspiro[3.4]octan-5-one, which was purified as the hydrochloride salt.

To a solution of DHA (7.5 g, 26.4 mmol, 1.0 eq) in DCM (300 mL) was added CDI (5.13 g, 31.7 mmol, 1.2 eq). The mixture was stirred at 25 °C for 2 h. Then 2,6-diazaspiro[3.4]octan-5-one (4.29 g, 26.4 mmol, 1.0 eq, HCl salt) and TEA (3.20 g, 31.7 mmol, 4.41 mL, 1.2 eq) was added to the above solution. The mixture was stirred at 25 °C for 12 h. LCMS indicated that the starting material was completely consumed and the desired mass was detected. The reaction mixture was concentrated under vacuum to give a residue which was purified by flash silica gel chromatography using a gradient of EtOAc/petroleum ether from 0/1 to 1/0. DHA 5-oxo-2,6-diazaspiro[3.4]octane-2-carboxylate (5.02 g, 11.5 mmol, 43.6% yield) was obtained as a white solid. M + H⁺ = 437.2 (LCMS); ¹H NMR (400 MHz, DMSO-*d₆*) δ 7.85 (s, 1H), 5.54 (s, 1H), 5.50 (br d, *J* = 9.6 Hz, 1H), 4.10 - 4.02 (m, 1H), 3.99 - 3.90 (m, 2H), 3.82 (br s, 1H), 3.16 (t, *J =* 6.7 Hz, 2H), 2.34 (br s, 2H), 2.29 - 2.14 (m, 2H), 2.00 (br d, *J =* 13.6 Hz, 1H), 1.86 - 1.78 (m, 1H), 1.67 - 1.58 (m, 2H), 1.53 (br t, *J* = 3.8 Hz, 1H), 1.45 (br d, *J* = 9.6 Hz, 2H), 1.30 (s, 4H), 1.22 - 1.13 (m, 1H), 1.01 - 0.93 (m, 1H), 0.89 (d, *J =* 6.3 Hz, 3H), 0.80 (br d, *J =* 7.0 Hz, 3H).

PT-30, DHA 5-oxo-2,6-diazaspiro[3.4]octane-2-carboxylate, (3*R*,5a*S*,6*R*,8a*S*,9*R*,12*S*,12a*R*)-decahydro-3,6,9-trimethyl-3,12-epoxy-12*H*-pyrano[4,3-*j*]--1,2-benzodioxepin-10-yl 5-oxo-2,6-diazaspiro[3.4]octane-2-carboxylate, was a white crystalline solid as prepared. Differential scanning calorimetry showed a broad peak starting at about 150 °C with peak at about 174 °C (no melting point was observed before decomposition), while thermogravimetric analysis showed about 0.9% weight loss by 150 °C. X-ray powder diffraction using Cu Kα radiation (λ = 1.5408 Å), showed sharp peaks characteristic of crystallinity, with the five largest peaks as follows, in °2θ (±0.2°) with approximate intensities in parentheses: 4.5 (70), 9.0 (100), 9.7 (46), 12.4 (46), and 13.6 (27). PT-30 had low solubility (<0.1 mg/mL) in aqueous acidic conditions and in simulated gastric fluid, and a solubility of about 0.7 mg/mL in fasted-state and fed-state simulated intestinal fluids. It was soluble in common organic solvents, with the greatest solubility in methanol and tetrahydrofuran. It was chemically stable without crystal form change when exposed to 25 °C/60%RH and 40 °C/75%RH for 1 week, and was slightly hygroscopic (0.5% water uptake from 40% to 95%RH).

### Examples

### Example 1: Antifibrotic effects in cardiac fibroblasts

Induced pluripotent stem cell-derived cardiac fibroblasts were constructed containing a fusion of the alpha-smooth muscle actin to the green fluorescent protein (ACTA2-GFP) construct. ACTA2 is a myofibroblast marker, such that the fibroblasts fluoresce with an intensity dependent on the extent of fibrosis. When these fibroblasts were activated with TGF-β, and simultaneously treated with the PT compounds, or the comparators dihydroartemisinin or artesunate, at various concentrations, for 48 hours and the fluorescence measured, they showed a dose-dependent reduction in fluorescence, with IC₅₀ for the PT compounds as follows, in µM: PT-1, 2.3; PT-2, 0.88; PT-4, 5.6; PT-13, 2.6; PT-14, 0.57; and artesunate, 2.8. The other PT compounds, including PT-28 and PT-30, also demonstrated antifibrotic efficacy in this model.

### Example 2: Pharmacokinetics in mice

The intravenous (IV) and oral (PO) pharmacokinetics of the PT compounds were measured in 6 - 10 week-old male C57BL/6J mice, using three mice/group. The test compounds were formulated in an appropriate water-based vehicle to give a clear solution for IV administration, or a clear solution or uniform suspension for PO administration. Animals were dosed within 4 hours of the preparation of the formulations; with the dose volume being determined by the animal's body weight measured on the morning of the dosing day. Blood samples (about 25 µL per time point) were taken at 5 and 15 minutes and 1, 3, and 5 hours after dosing, into pre-chilled EDTA-K2 tubes and kept on wet ice until centrifugation. The tubes were centrifuged at 3200×g at about 4° C for 10 minutes, and the plasma analyzed by LC-MS/MS. The results are given in the table below.

| Compound ID | IV | | | | PO | | |
|---|---|---|---|---|---|---|---|
| | T_{1/2} (h) | V_{D,ss} (L/kg) | Clearance (mL/min/kg) | AUC₀₋ₗₐₛₜ (µg.h/mL) | T_{1/2} (h) | AUC₀₋ₗₐₛₜ (µg.h/mL) | Bioavailability (%) |
| PT-1 | 0.13 | 9.0 | 1280 | 0.1 | 0.37 | 0.02 | 1.3 |
| PT-2 | 0.10 | 1.3 | 140 | 1.2 | | 3.2 | 27 |
| PT-4 | 0.11 | 2.0 | 280 | 0.6 | 0.18 | 0.5 | 11 |
| PT-13 | 0.12 | 0.9 | 76 | 2.2 | 0.69 | 14.1 | 64 |
| PT-14 | 0.18 | 1.1 | 75 | 2.2 | 0.66 | 20.5 | 92 |
| PT-20 | 0.24 | 0.8 | 69 | 2.4 | 0.53 | 17.7 | 73 |
| PT-24 | 0.26 | 2.2 | 77 | 2.2 | 1.12 | 24.3 | 123 |
| PT-25 | 0.27 | 1.3 | 74 | 2.2 | 1.89 | 3.7 | 17 |
| PT-27 | 0.25 | 1.0 | 47 | 3.3 | 1.30 | 14.4 | 41 |
| PT-28 | 0.24 | 0.8 | 38 | 4.2 | 0.48 | 48.8 | 112 |
| PT-29 | 0.13 | 0.9 | 82 | 2.0 | 1.08 | 6.8 | 34 |
| PT-30 | 0.22 | 0.6 | 30 | 5.3 | 0.34 | 40.9 | 73 |

### Example 3: Plasma protein binding

Plasma protein binding assays were conducted in triplicate for compounds PT-2, PT-14, PT-28, and PT-30, with artesunate, DHA, and warfarin as comparators. The results are given in the table below.

| Compound ID | Mouse | | Human | |
|---|---|---|---|---|
| | % unbound (SD) | % recovery (SD) | % unbound (SD) | % recovery (SD) |
| PT-2 | 8.1 (NC) | 82.3 (19.5) | 5.4 (0.3) | 91.4 (0.9) |
| PT-14 | 10.5 (0.8) | 100.3 (0.7) | 29.0 (0.4) | 100.8 (2.7) |
| PT-28 | 8.0 (2.0) | 101.0 (9.9) | 19.2 (1.1) | 89.4 (11.9) |
| PT-30 | 7.9 (2.2) | 87.6 (4.2) | 45.5 (NC) | 84.9 (9.4) |
| artesunate | 27.8 (6.5)* | 53.4 (8.7) | 23.3 (2.1) | 98.0 (2.2) |
| DHA | 10.0 (07) | 105.0 (2.4) | 10.8 (0.2) | 102.5 (5.7) |
| warfarin | 0.7 (0.0) | 94.5 (2.8) | 0.3 (0.0) | 91.1 (1.6) |

| | | | | |
|---|---|---|---|---|
| NC: not calculated; *: % unbound value may be unreliable due to low recovery | | | | |

### Example 4: HEK Blue-TLR4 assay (MD2 pathway)

HEK Blue-TLR4 cells were plated in detection media, preincubated with vehicle or PT-2, PT-14, or PT-30 dissolved in vehicle (with artesunate, DHA, and artemisinin comparators) for 1 h, then co-stimulated with 10ng/mL lipopolysaccharide. Reporter activity was read after 20 hours at 655nm in a spectrophotometer. The PT compounds were active in this assay, showing that they inhibit the MD2 signaling pathway.

### Example 5: Other assays of PT-30

PT-30 demonstrated antifibrotic efficacy in primary pulmonary fibroblasts, measuring the effect on acta2, col1a1, and CTGF; demonstrated antifibrotic efficacy in the CCl₄ injury mouse liver model by oral administration; and demonstrated antifibrotic efficacy in the bleomycin injury mouse skin model by topical administration (50 µL, 100 µM in 0.1%DMSO/PBS).

### Example 6: Prophetic human example in PBC using PT-30

Trial subjects are adult, male or female, with a diagnosis of primary biliary cholangitis (PBC) by at least two of the following three criteria: (a) a history of alkaline phosphatase (ALP) above the upper limit of normal (ULN) for at least six months, (b) positive anti-mitochondrial antibody titers > 1/40 on immunofluorescence or M2 positive by enzyme linked immunosorbent assay or positive PBC-specific antinuclear antibodies, and (c) documented liver biopsy result consistent with PBC, on a stable and recommended dose of UDCA for the past twelve months or UDCA intolerant, and ALP ≥ 1.67 × ULN. Exclusion criteria include AST or ALT ≥ 3 × ULN, total bilirubin (TBIL) ≥ 2 × ULN, autoimmune hepatitis or a history of chronic viral hepatitis, PSC, the current use of fibrates or simvastatin, the use of colchicine, methotrexate, azathioprine, or systemic steroids in the previous two months, the use of an experimental treatment for PBC, and the use of an experimental or unapproved immunosuppressant. The primary study endpoint is decrease in ALP, and the secondary endpoint is the responder rate for subjects achieving ALP < 1.67 × ULN and total bilirubin within normal limit, and > 15% decrease in ALP. Additional secondary endpoints are changes in GGT, TBIL, and 5'-nucleotidase, which are other recognized biochemical markers of PBC. Subjects are randomized to receive either placebo, 10 mg/day, 30 mg/day, 50 mg/day, 100mg/day, 200 mg/day, or 400 mg/day of PT-30 orally once/day in tablet form for 12 weeks. The subjects show a dose-dependent reduction in ALP, indicating a treatment effect of the PT-30 administration on PBC fibrosis.

## Claims

1. A compound of any one of the formulae PT-1, PT-2, PT-4, PT-13, PT-14, PT-20, PT-23 to PT-25, and PT-27 to PT-30: 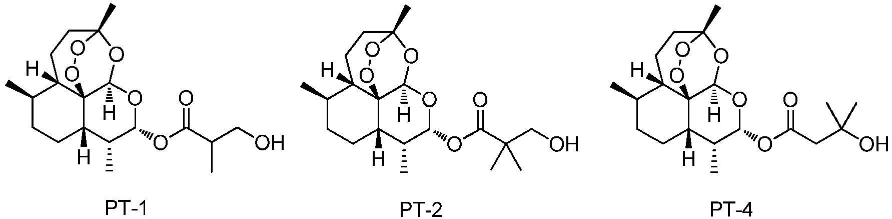 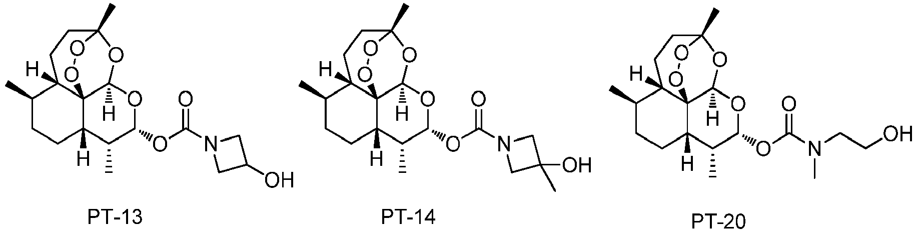 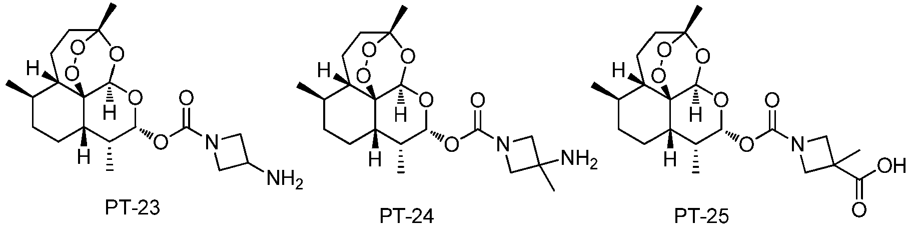 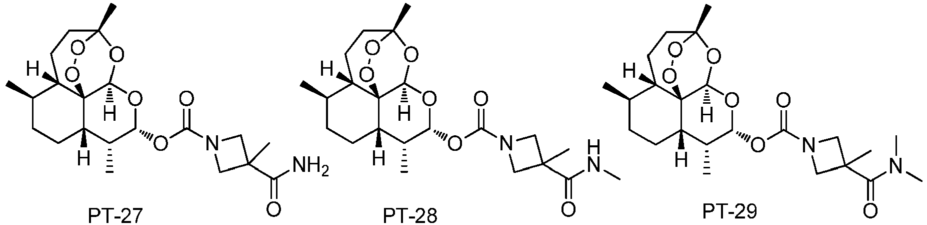 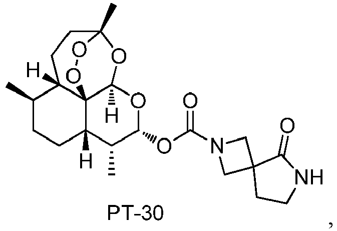 or a salt, especially a pharmaceutically acceptable salt, thereof.

2. The compound of claim 1 that is a compound of any one of the formulae PT-2, PT-14, PT-28, and PT-30, or a salt, especially a pharmaceutically acceptable salt, thereof.

3. The compound of claim 1 that is a compound of formula PT-14 or PT-30, or a salt, especially a pharmaceutically acceptable salt, thereof.

4. The compound of claim 3 that is a compound of formula PT-14, or a salt, especially a pharmaceutically acceptable salt, thereof.

5. The compound of claim 4 that is a compound of formula PT-14.

6. The compound of claim 3 that is a compound of formula PT-30, or a salt, especially a pharmaceutically acceptable salt, thereof.

7. The compound of claim 6 that is a compound of formula PT-30.

8. The compound of claim 1 that is a compound of any one of the formulae PT-1, PT-2, and PT-4, or a salt, especially a pharmaceutically acceptable salt, thereof.

9. The compound of claim 1 that is a compound of one of the formulae PT-13, PT-14, PT-20, PT-23 to PT-25, and PT-27 to PT-30, or a salt, especially a pharmaceutically acceptable salt, thereof.

10. A pharmaceutical composition comprising a compound of any one of claims 1 to 9.

11. A compound of any one of claims 1 to 9, or a pharmaceutical composition of claim 10, for use as a medicament.

12. A compound of any one of claims 1 to 9, or a pharmaceutical composition of claim 10, for use in treating a fibrotic disease.

13. The compound or pharmaceutical composition for use of claim 12, where the fibrotic disease is a systemic fibrotic disease.

14. The compound or pharmaceutical composition for use of claim 13, where the systemic fibrotic disease is systemic sclerosis, multifocal fibrosclerosis (IgG4-associated fibrosis), nephrogenic systemic fibrosis, or sclerodermatous graft-versus-host disease.

15. The compound or pharmaceutical composition for use of claim 12, where the fibrotic disease is an organ-specific fibrotic disease.

16. The compound or pharmaceutical composition for use of claim 15, where the organ-specific fibrotic disease is cardiac fibrosis, kidney fibrosis, pulmonary fibrosis, liver and portal vein fibrosis, radiation-induced fibrosis, bladder fibrosis, intestinal fibrosis, pancreatic fibrosis, peritoneal sclerosis, diffuse fasciitis, localized scleroderma, keloids, Dupuytren's disease, Peyronie's disease, myelofibrosis, or oral submucous fibrosis.

## Patentansprüche

1. Verbindung einer beliebigen der Formeln PT-1, PT-2, PT-4, PT-13, PT-14, PT-20, PT-23 bis PT-25 und PT-27 bis PT-30: 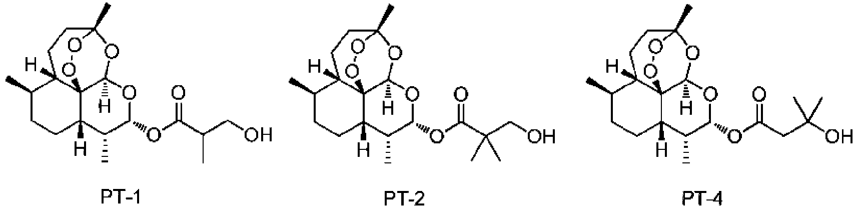 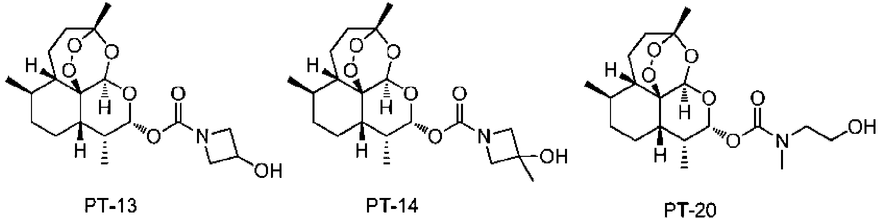 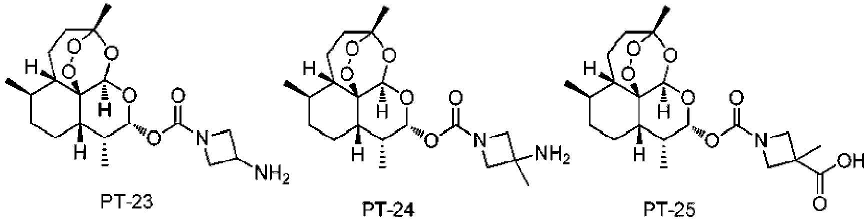 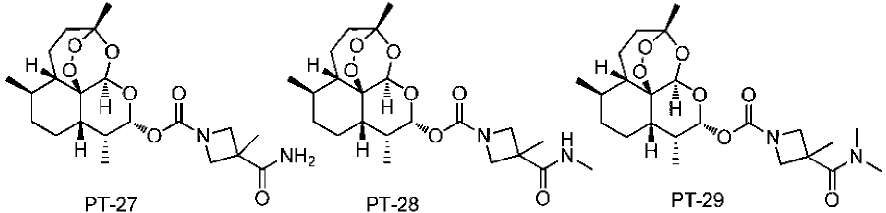 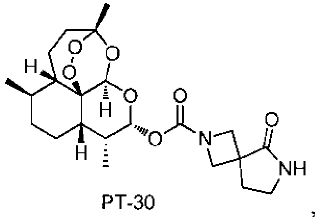 oder ein Salz, insbesondere ein pharmazeutisch annehmbares Salz, davon.

2. Verbindung nach Anspruch 1, die eine Verbindung einer beliebigen der Formeln PT-2, PT-14, PT-28 und PT-30 ist, oder ein Salz, insbesondere ein pharmazeutisch annehmbares Salz, davon.

3. Verbindung nach Anspruch 1, die eine Verbindung der Formel PT-14 oder PT-30 ist, oder ein Salz, insbesondere ein pharmazeutisch annehmbares Salz, davon.

4. Verbindung nach Anspruch 3, die eine Verbindung der Formel PT-14 ist, oder ein Salz, insbesondere ein pharmazeutisch annehmbares Salz, davon.

5. Verbindung nach Anspruch 4, die eine Verbindung der Formel PT-14 ist.

6. Verbindung nach Anspruch 3, die eine Verbindung der Formel PT-30 ist, oder ein Salz, insbesondere ein pharmazeutisch annehmbares Salz, davon.

7. Verbindung nach Anspruch 6, die eine Verbindung der Formel PT-30 ist.

8. Verbindung nach Anspruch 1, die eine Verbindung einer beliebigen der Formeln PT-1, PT-2 und PT-4 ist, oder ein Salz, insbesondere ein pharmazeutisch annehmbares Salz, davon.

9. Verbindung nach Anspruch 1, die eine Verbindung einer der Formeln PT-13, PT-14, PT-20, PT-23 bis PT-25 und PT-27 bis PT-30 ist, oder ein Salz, insbesondere ein pharmazeutisch annehmbares Salz, davon.

10. Pharmazeutische Zusammensetzung, die eine Verbindung nach einem der Ansprüche 1 bis 9 umfasst.

11. Verbindung nach einem der Ansprüche 1 bis 9 oder pharmazeutische Zusammensetzung nach Anspruch 10 zur Verwendung als Medikament.

12. Verbindung nach einem der Ansprüche 1 bis 9 oder pharmazeutische Zusammensetzung nach Anspruch 10 zur Verwendung bei der Behandlung einer fibrotischen Erkrankung.

13. Verbindung oder pharmazeutische Zusammensetzung nach Anspruch 12, wobei die fibrotische Erkrankung eine systemische fibrotische Erkrankung ist.

14. Verbindung oder pharmazeutische Zusammensetzung nach Anspruch 13, wobei die systemische fibrotische Erkrankung systemische Sklerose, multifokale Fibrosklerose (IgG4-assoziierte Fibrose), nephrogene systemische Fibrose oder sklerodermiforme Graft-versus-Host-Erkrankung ist.

15. Verbindung oder pharmazeutische Zusammensetzung nach Anspruch 12, wobei die fibrotische Erkrankung eine organspezifische fibrotische Erkrankung ist.

16. Verbindung oder pharmazeutische Zusammensetzung nach Anspruch 15, wobei die organspezifische fibrotische Erkrankung Herzfibrose, Nierenfibrose, Lungenfibrose, Leber- und Portalvenenfibrose, strahleninduzierte Fibrose, Blasenfibrose, Darmfibrose, Pankreasfibrose, Peritonealsklerose, diffuse Fasziitis, lokalisierte Sklerodermie, Keloide, Morbus Dupuytren, Morbus Peyronie, Myelofibrose oder orale submuköse Fibrose ist.

## Revendications

1. Composé de l'une quelconque des formules PT-1, PT-2, PT-4, PT-13, PT-14, PT-20, PT-23 à PT-25 et PT-27 à PT-30 : 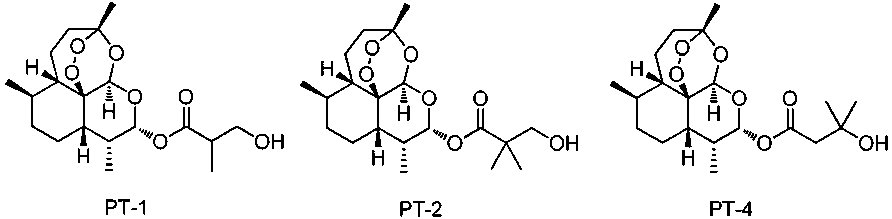 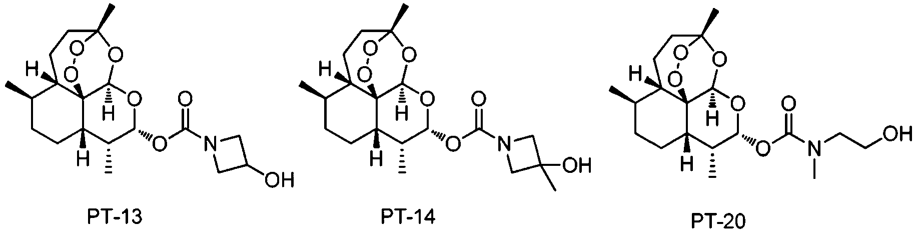 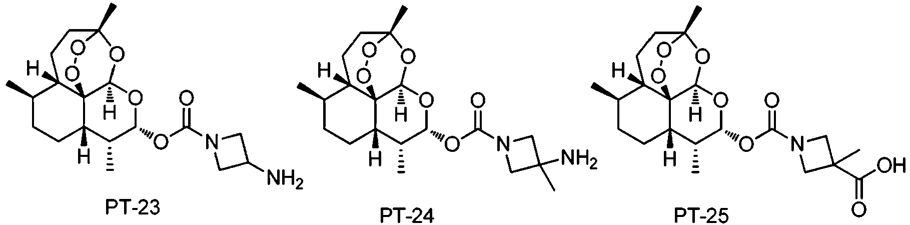 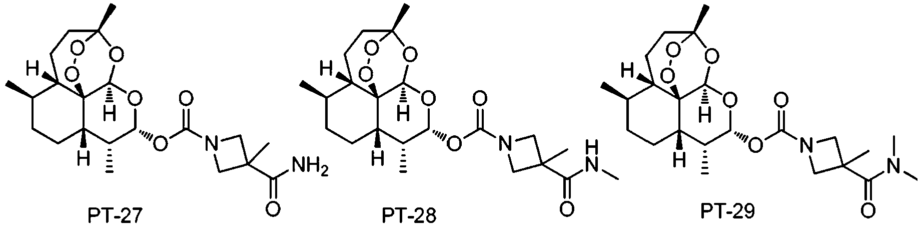 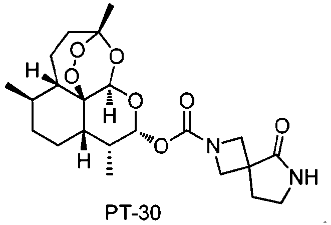 ou un sel, en particulier un sel pharmaceutiquement acceptable, de celui-ci.

2. Composé selon la revendication 1, qui est un composé de l'une quelconque des formules PT-2, PT-14, PT-28 et PT-30, ou un sel, en particulier un sel pharmaceutiquement acceptable, de celui-ci.

3. Composé selon la revendication 1, qui est un composé de formule PT-14 ou PT-30, ou un sel, en particulier un sel pharmaceutiquement acceptable, de celui-ci.

4. Composé selon la revendication 3, qui est un composé de formule PT-14, ou un sel, en particulier un sel pharmaceutiquement acceptable, de celui-ci.

5. Composé selon la revendication 4, qui est un composé de formule PT-14.

6. Composé selon la revendication 3, qui est un composé de formule PT-30, ou un sel, en particulier un sel pharmaceutiquement acceptable, de celui-ci.

7. Composé selon la revendication 6, qui est un composé de formule PT-30.

8. Composé selon la revendication 1, qui est un composé de l'une quelconque des formules PT-1, PT-2 et PT-4, ou un sel, en particulier un sel pharmaceutiquement acceptable, de celui-ci.

9. Composé selon la revendication 1, qui est un composé de l'une quelconque des formules PT-13, PT-14, PT-20, PT-23 à PT-25 et PT-27 à PT-30, ou un sel, en particulier un sel pharmaceutiquement acceptable, de celui-ci.

10. Composition pharmaceutique comprenant un composé selon l'une quelconque des revendications 1 à 9.

11. Composé selon l'une quelconque des revendications 1 à 9, ou composition pharmaceutique selon la revendication 10, pour utilisation en tant que médicament.

12. Composé selon l'une quelconque des revendications 1 à 9, ou composition pharmaceutique selon la revendication 10, pour utilisation dans le traitement d'une maladie fibrotique.

13. Composé ou composition pharmaceutique pour utilisation selon la revendication 12, la maladie fibrotique étant une maladie fibrotique systémique.

14. Composé ou composition pharmaceutique pour utilisation selon la revendication 13, la maladie fibrotique systémique étant la sclérose systémique, la fibrosclérose multifocale (fibrose associée à IgG4), la fibrose systémique néphrogénique, ou la maladie du greffon contre l'hôte sclérodermique.

15. Composé ou composition pharmaceutique pour utilisation selon la revendication 12, la maladie fibrotique étant une maladie fibrotique spécifique à un organe.

16. Composé ou composition pharmaceutique pour utilisation selon la revendication 15, la maladie fibrotique spécifique à un organe étant la fibrose cardiaque, la fibrose rénale, la fibrose pulmonaire, la fibrose du foie et de la veine porte, la fibrose induite par rayonnement, la fibrose vésicale, la fibrose intestinale, la fibrose pancréatique, la sclérose péritonéale, la fasciite diffuse, la sclérodermie localisée, les chéloïdes, la maladie de Dupuytren, la maladie de Peyronie, la myélofibrose ou la fibrose sous-muqueuse orale.
